(19) Europäisches Patentamt
European Patent Office
Office européen des brevets

(11) Veröffentlichungsnummer: **0 009 200**
**B1**

(12) **EUROPÄISCHE PATENTSCHRIFT**

(21) Anmeldenummer: **79103383.0**

(51) Int. Cl.³: **C 07 C 121/43**

(22) Anmeldetag: **11.09.79**

(54) **Verfahren zur Herstellung von N,N-Dimethylaminoacetonitril**

(30) Priorität: **21.09.78 DE 2840990**

(43) Veröffentlichungstag der Anmeldung:
**02.04.80 Patentblatt 80/07**

(45) Bekanntmachung des Hinweises auf die Patenterteilung:
**14.01.81 Patentblatt 81/02**

(84) Benannte Vertragsstaaten:
**BE CH DE FR GB IT NL**

(56) Entgegenhaltungen:
**DE - A1 - 2 503 582**
**DE - A1 - 2 555 769**
**GB - A - 1 182 405**

**HOUBEN-WEYL, "METHODEN DER ORGANISCHEN CHEMIE," Bd. VIII, Sauerstoffverbindungen III 1952 Georg Thieme Verlag Stuttgart Seiten 274 bis 277**

(73) Patentinhaber: **BAYER AG**
**Zentralbereich Patente, Marken und Lizenzen**
**D - 5090 Leverkusen 1, Bayerwerk (DE)**

(72) Erfinder: **Lenthe, Manfred, Dr.**
**Michaelshöhe 40**
**D - 5068 Odenthal-Neschen (DE)**
**Dankert, Gerhard, Dr.**
**Arthur-Hantzsch-Strasse 44**
**D-5000 Köln 80 (DE)**

**EP 0 009 200 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von N,N-Dimethylaminoacetonitril

Die vorliegende Erfindung betrifft ein neues Verfahren sur Herstellung von N,N-Dimethylaminoacetonitril, welches bekanntermaßen als Zwischenprodukt für die Synthese von pestiziden Wirkstoffen verwendet werden kann.

Die Herstellung von N,N-Dimethylaminoacetonitril aus Dimethylamin, Blausäure und Formaldehyd nach einer lange bekannten Reaktion (Typ der sogenannten "Mannich-Reaktion," vgl. "Organikum," VEB Deutscher Verlag der Wissenschaften, Berlin, 8.Aufl. (1968), Seite 447ff.) gelingt bekanntlich in hohen Ausbeuten, wenn Blausäure in wasserfreier Form oder als Alkalisalz mit Salzsäure eingesetzt wird (vgl. J. Am. Chem. Soc. *68* (1946), 1607/08; Liebigs Ann. Chem., *279*, 43 (1894); Deutsche Patentschrift 694 808. Die Gewinnung des N,N-Dimethylaminoacetonitrils aus den wasserhaltigen Reaktionsgemischen kann durch Extraktion mit 1,2-Dichlorbenzol erfolgen (vgl. Deutsche Patentanmeldung P 27 48 964.1).

Diese bekannten Verfahren haben den Nachteil der Verwendung wasserfreier Blausäure, die strengen Transport-, Lager- und Verarbeitungsauflagen genügen muß, oder, bei Verwendung der Salze der Blausäure (Natriumcyanid, Kaliumcyanid), des Salzanfalls sowie der Abtrennung großer Wassermengen. Diese Nachteile erschweren oder verhindern die wirtschaftliche Nutzung der bekannten Verfahren.

Es wurde nun gefunden, daß man das bekannte N,N-Dimethylaminoacetonitril der Formel

$$(CH_3)_2N - CH_2 - CN \qquad (I)$$

in besonders einfacher Weise dann erhält, wenn man Acetoncyanhydrin der Formel

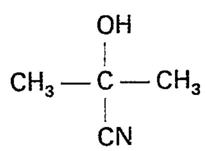

(II)

mit Dimethylamin und Formaldehyd im Temperaturbereich zwischen −10 und +120°C, gegebenenfalls in Gegenwart basischer Katalysatoren, umsetzt.

Es ist als ausgesprochen überraschend zu beseichnen, daß man bei Verwendung von Acetoncyanhydrin anstelle von freier Blausäure in einfacher Weise und in hoher Ausbeute das gewünschte Endproduckt I erhält.

Das erfindungsgemäße Verfharen weist eine Reihe von Vorsteilen auf. So kann Acetoncyanhydrin im Gegensatz zu wasserfreier Blausäure problemlos gefördert, gelagert und eingesetzt werden, da es infolge des hohen Siedepunktes (der Siedepunkt liegt bei Kp 81°C/20 mbar) eine wesentlich geringere Gefährdung hervorruft als Blausäure. Zusätzlich wird durch diese Chemikalie kein Wasser in den Prozeß eingeschleppt, das in der nachfolgenden Extraktion mit einem entsprechend höheren Aufwand an Extraktionsmittel wieder ausgeschleust werden müßte. Das Verfahren ist besonders umweltfreundlich, da es keine Salze erzeugt. Es wird somit ein technischer Fortschritt erzielt.

Der Reaktionsablauf kann durch das folgende Formelschema wiedergegeben werden:

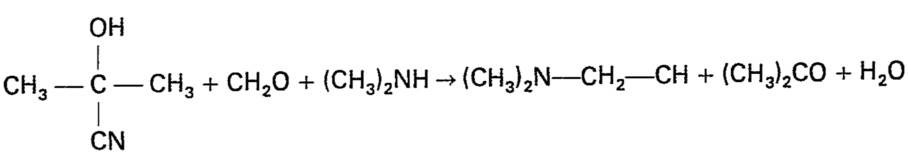

Das als Vorprodukt benötigte Acetoncyanhydrin der Formel II ist ein großtechnisch aus Blausäure und Aceton erhältliches Handelsprodukt (bekannt auch als Vorprodukt von Methacrylsäureester). Die anderen Vorprodukte Formaldehyd und Dimethylamin sind ebenfalls leicht zugängliche, bekannte Handelsprodukte. Formaldehyd kann in Form seiner wäßrigen Lösung (z.B. als sogenannte 30 %ige Formalinlösung) oder als Paraformaldehyd eingesetzt werden.

Die Anwendung von Acetoncyanhydrin zur Herstellung von N,N-Dimethylaminoacetonitril gestaltet sich besonders einfach, da eine vorangehende Spaltung des Cyanhydrins in Aceton und Blausäure nicht erforderlich ist, sondern die Substanz in der Lieferform eingesetzt werden kann; auch eventuell vorhandene Stabilisierungszusätze brauchen nicht entfernt zu werden.

Die Geschwindigkeit der Reaktion kann durch Zugabe geringer Mengen basischer Substanzen als Hilfsstoffe katalysiert werden. Die Reaktion läuft besonders schnell ab, wenn man sie bei pH-Werten zwischen 12 und 4, vorzugsweise zwischen 9 und 6, durchführt. Als basische Katalysatoren können

Metallhydroxide, bevorzugt von Metallen aus der 1. und 2. Hauptgruppe des Periodensystems der Elemente, verwendet werden. Der Katalysator kann z.B. dem Reaktionsgemisch in Form wäßriger Alkalilauge oder festen Alkalihydroxides zugeführt werden, die Alkalihydroxide sind gegebenenfalls auch durch andere Hydroxide ersetzbar. Weiterhin können auch basische Ionenaustauscher als Katalysatoren Verwendung finden.

Die Umsetzung wird bei Temperaturen zwischen −10 und +120°C, vorzugsweise zwischen +20 und 80°C vorgenommen.

Das erfindungsgemäße Verfahren kann unter Vakuum wie auch unter erhöhtem Druck durchgeführt werden. Vorzugsweise wird jedoch bei Normaldruck gearbeitet.

Bei der Durchführung des erfindungsgemäßen Verfahrens setzt man auf ein Mol Acetoncyanhydrin je ein Mol Formaldehyd (oder die äquivalente Menge Paraformaldehyd bzw. Formalinlösung) und Dimethylamin in reiner Form oder in Lösungsmittel wie z.B. Wasser gelöst, ein. Zur besseren Ausnutzung teurer Chemikalien ist jedoch auch der überstöchiometrische Einsatz einer oder zweier Komponenten möglich, ohne daß dadurch die Ausbeute herabgesetzt wird. Die Reihenfolge des Zusammengebens der Reaktionskomponenten kann in dem erfindungsgemäßen Herstellungsverfahren weitgehend ohne Einfluß auf das Ergebnis der Umsetzung variiert werden. So sind folgende Varianten durchführbar:

1. Acetoncyanhydrin mit Dimethylamin vermischen, danach mit Formaldehyd umsetzen und Aceton abdestillieren.
2. Formaldehyd mit Dimethylamin umsetzen, Acetoncyanhydrin zuführen und Aceton abdestillieren.
3. Acetoncyanhydrin und Formaldehyd mischen, Aceton abdestillieren und den Rückstand mit Dimethylamin umsetzen.

Die unter (1) genannte Verfahrensform ist im Gegensatz zur Verwendung freier Blausäure unkritisch, da in der basischen Lösung Acetoncyanhydrin einige Zeit stabil ist, Blausäure jedoch sofort unter Polymerisation reagiert.

Das bei der Reaktion entstehende Aceton kann leicht destillativ aus dem Reaktionsgemisch entfernt werden. Bei einer Extraktion mit einem geeigneten Extraktionsmittel (Siehe unten) ist es jedoch auch möglich, das in das Extraktionsmittel gehende Aceton als Vorlauf bei der Abtreibung des Dimethylaminoacetonitrils zurückzugewinnen und für die Herstellung des Acetoncyanhydrins oder für andere Zwecke einzusetzen.

Die Schnelligkeit, mit der die Reaktion abläuft, läßt neben der diskontinuierlichen auch mit geringen Verweilzeiten die kontinuierliche Verfahrensweise zu. So ist es unter anderem möglich, die Umsetzungen unter synchroner destillativer Abtrennung von Aceton in einer kontinuierlich betriebenen Destillationskolonne durchzuführen.

Die Aufarbeitung wird wie folgt vorgenommen:

Nach der Reaktion muß das N-N-Dimethylaminoacetonitril aus dem Gemisch mit Wasser (Reaktions- wie auch eingebrachtes Wasser) isoliert werden. Dieser Schritt kann besonders günstig in einer Flüssig-Flüssig-Extraktion mit 1,2-Dichlorbenzol als Extraktionsmittel durchgeführt werden. Die Extraktion wird im mehrstufigen Gegenstrombetrieb mit 2,5 bis 5 Gewichtsteilen 1,2-Dichlorbenzol auf 1 Gewichtsteil Dimethylaminoacetonitril/Wasser-Gemisch bei Temperaturen zwischen 15 und 30°C gefahren. Aus dem Extrakt wird Dimethylaminoacetonitril in besonders reiner Form durch Destillation gewonnen (Gegenstand der Deutschen Patentanmeldung P 27 48 964.1).

Bei geringen Wassermengen im Reaktionsgemisch (im Grenzfall nur Reaktionswasser mit ca. 18 Gew.-%) ist auch die Abtrennung des Wassers mit z.B. Toluol oder einem anderen Stoff möglich, der mit Wasser ein heterogenes Azeotrop mit einem Siedepunkt unter dem Siedepunkt des homogenen Dimethylaminoacetnitril/Wasser-Azeotrops bildet. Dieses Auskreisen von Wasser wird bei Drucken zwischen 100 und 2000 mbar, vorzugsweise zwischen 900 und 1100 mbar, durchgeführt. Die zum Abtrennen des Wassers verwendete Substanz wird anschließend von N,N-Dimethylaminoacetonitril wieder durch Destillation abgetrennt.

Das so erfindungsgemäß herstellbare N,N-Dimethylaminoacetonitril und die daraus durch Verseifung zu gewinnende $\alpha$-Aminocarbonsäure sind wichtige Zwischenprodukte für die Herstellung von Farbstoffen, Textilhilfsmitteln, pharmazeutischen Produckten und Wirkstoffen für de Pflazenschutz. So kann z.B. aus dem obengenannten Nitril durch Umsetzung mit Chlor in einer Umlagerungsreaktion das Tetrachloräthylen-bis-isocyaniddichlorid gewonnen werden. Die letztgenannte Verbindung ergibt mit Fluorwasserstoff das N,N'-Bis-(trifluormethyl)-tetrafluoräthyendiamin, aus letzterem erhält man mit N-methyl-N'-(4-chlorphenyl)-thioharnstoff in Gegenwart von Natriumfluorid unter Abspaltung von 4 Molen Fluorwasserstoff in einer Ringschlußreaktion das literaturbekannte Pflanzenschutz-Fungizid 2-Methylimino-3-(4'-chlorphenyl)-4,5-bis-(trifluormethylimino)-thiazolidin (vgl. DE—OS 2 210 882, sowie DE—OS 2 062 348 bzw. US—PS 3 895 020).

Beispiel 1

In 200,2 g Formalinlösung mit einem Gehalt von 30 Gew.-% (Ca. 60 g oder 2 Mol) Formaldehyd wurden 90,0 g (2 Mol) Dimethylamin bei einer Temperatur von maximal + 40°C eingeleitet. Zu dieser Lösung wurden 170 g (2 Mol) Acetoncyanhydrin gegeben. In einer leicht exothermen Reaktion stellte sich ein

pH-Wert von 7,5 ein. Nach Erhitzen auf 77°C begann das Reaktionsgemisch zu sieden und über eine kurze Destillationskolonne konnte eine Fraktion von 125 g mit einem Gehalt von 95 % Aceton bei einer Übergangstemperatur von 55 bis 60°C bei Normaldruck gewonnen werden.

Das zurückbleibende Sumpfgemisch enthielt nach gaschromatographischer Ananlyse 46 Gew.-% Dimethylaminoacetonitril und nach fünfmaliger Extraktion mit je 100 ml Diäthyläther konnten nach Abdampfen des Äthers 155 g N,N-Dimethylaminoacetonitril vom Kp 78°C/133 mbar, entsprechend einer Ausbeute von 92%, erhalten werden.

Beispiel 2

30 g (1 Mol) Paraformaldehyd werden in einer Lösung von 170 g (2 Mol) Acetoncyanhydrin und 90 g (2 Mol) Dimethylamin suspendiert. Nach Eintragung von 4 ml 50 %iger Kalilauge werden weitere 30 g (1 Mol) Paraformaldehyd so zugegeben, daß die Temperatur 50°C nicht überschreitet. Der pH-Wert stellt sich dabei auf 11 ein.

Nach Abdestillation von 118 g Aceton bleibt ein Sumpf zurück, der nach dreimaligen Ausschütteln mit je 200 g o-Dichlorbenzol und destillativer Trennung der Extraktionsphase 161 g N,N-Dimethylaminoacetonitril vom Kp 78°C/100 bar ergibt. Die Ausbeute beträgt 96 %.

Beispiel 3

170 g (2 Mol) Acetoncyanhydrin und 200 g 30 %ige Formalinlösung (2 Mol Formaldehyd) wurden in einem 500 ml Rührbehälter unter Kühlung vermischt. Diese Lösung wurde mit 1 ml 50 %iger Kalilauge versetzt und erwärmt. Bei einem Rücklaufverhältnis von 2 : 1 wurden über einen kurzen Destillationsaufsatz mit einer Sumpftemperature von 77°C und einer Kopftemperatur von 57°C 110 g Aceton mit einem Gehalt von 97 % abdestilliert.

In der verbleidenden Sumpf wurden bei 10°C 90 g (2 Mol) Dimethylamin langsam eingeleitet. Nach extraktiver Behandlung des Gemisches gamäß Beispiel 2 wurden 160 g N,N-Dimethylaminoacetonitril erhalten, was einer Ausbeute von 95 % der Theorie entspricht.

**Patentansprüche**

1. Verfahren zur Herstellung von N,N-Dimethylaminoacetonitril der Formel

$$(CH_3)_2N — CH_2 — CN$$

dadurch gekennzeichnet, daß man Acetoncyanhydrin der Formel

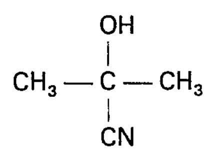

mit Dimethylamin und Formaldehyd im Temperaturbereich zwischen −10 und +120°C, gegebenenfalls in Gegenwart basischer Katalysatoren, umsetzt.

2. Verfahren nach Anspruch 1, dadurch gekennzeichnet, daß man die Umsetzung im Temperaturbereich zwischen +20 und 80°C durchführt.

3. Verfahren nach Anspruch 1, dadurch gekennzeichnet, daß man die Umsetzung im pH-Bereich zwischen 12 und 4 durchführt.

4. Verfahren nach Ansprüchen 1 und 3, dadurch gekennzeichnet daß man die Umsetzung im pH-Bereich zwischen 9 und 6 durchführt.

5. Verfahren nach Ansprüchen 1 und 5, dadurch gekennzeichnet, daß man als Katalysatoren Metallhydroxide der 1. und 2. Hauptgruppe des Periodensystems der Elemente verwendet.

6. Verfahren nach Ansprüchen 1 und 5, dadurch gekennzeichnet, daß man als Katalysatoren basische Ionenaustauscher verwendet.

**Revendications**

1. Procédé de fabrication de N,N-diméthylaminoacétonitrile de formule

$$(CH_3)_2N—CH_2—CN$$

caractérisé en ce qu'on fait réagir de l'acétonecyanhydrine de formule

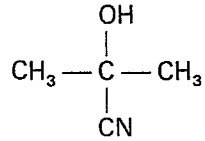

avec de la diméthylamine et de la formaldéhyde dans l'intervalle de température de — 10 à + 120°C, éventuellement en présence de catalyseurs basiques.

2. Procédé selon la revendication 1, caractérisé en ce qu'on exécute la réaction dans l'intervalle der température de +20 à 80°C.

3. Procédé selon la revendication 1, caractérisé en ce qu'on exécute la réaction dans l'intervalle de pH de 12 à 4.

4. Procédé selon l'une quelconque des revendications 1 et 3, caractérisé en ce qu'on exécute la réaction dans l'intervalle de pH de 9 à 6.

5. Procédé selon l'une quelconque des revendications 1 et 5 (selon la revendication 1), caractérisé en ce qu'on utilise comme catalyseurs des hydroxydes métalliques du premier et du second groupe principal du système périodique des éléments.

6. Procédé selon l'une quelconque des revendications 1 et 5 (selon la revendication 1), caractérisé en ce qu'on utilise comme catalyseurs des échangeurs d'ions basiques.

**Claims**

1. Process for the preparation of N,N-dimethylaminoacetonitrile of the formula

$$(CH_3)_2N — CH_2 — CN$$

characterised in that acetone cyanohydrin of the formula

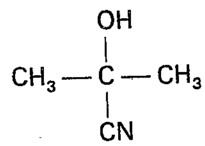

is reacted with dimethylamine and formaldehyde in the temperature range between — 10 and + 120°C, optionally in the presence of basic catalysts.

2. Process according to Claim 1, characterised in that the reaction is carried out in the temperature range between + 20 and 80°C.

3. Process according to Claim 1, characterised in that the reaction is carried out in the pH range between 12 and 4.

4. Process according to Claims 1 and 3, characterised in that the reaction is carried out in the pH range between 9 and 6.

5. Process according to Claims 1 and 5 (according to Claim 1), characterised in that metal hydroxides of the 1st and 2nd main group of the periodic system of the elements are used as catalysts.

6. Process according to Claims 1 and 5 (according to Claim 1), characterised in that basic ion exchangers are used as catalysts.